# EUROPÄISCHE PATENTSCHRIFT

(11) **EP 1 169 289 B1**
(45) Veröffentlichungstag und Bekanntmachung des Hinweises auf die Patenterteilung: **29.10.2003**
(21) Anmeldenummer: 00907558.1
(22) Anmeldetag: 12.02.2000
(51) Int. Cl.: C07C 63/331, C07C 51/00

(54) **VERFAHREN ZUR HERSTELLUNG VON DERIVATEN DER BIPHENYL-2-CARBONSÄURE**
METHOD FOR PRODUCING DERIVATIVES OF BIPHENYL-2-CARBOXYLIC ACID
PROCEDE POUR PRODUIRE DES DERIVES D'ACIDE BIPHENYL-2-CARBOXYLIQUE

(30) Priorität: 26.02.1999 DE 19908504
(43) Veröffentlichungstag der Anmeldung: 09.01.2002
(73) Patentinhaber: Boehringer Ingelheim Pharma GmbH & Co.KG, 55218 Ingelheim am Rhein (DE)
(72) Erfinder: SCHNEIDER, Heinrich, D-55218 Ingelheim am Rhein (DE)
(86) Internationale Anmeldenummer: EP0001162
(87) Internationale Veröffentlichungsnummer: WO00051961

(56) Entgegenhaltungen:
- EP-A- 0 059 983
- DATABASE WPI Section Ch, Week 197714 Derwent Publications Ltd., London, GB; Class B05, AN 1977-24139Y XP002141102 & JP 52 023058 A (SANKYO CO LTD), 21. Februar 1977 (1977-02-21)

## Beschreibung

Die Erfindung betrifft ein im technischen Maßstab anwendbares Verfahren zur Herstellung von Biphenyl-2-carbonsäure-Derivaten (I) 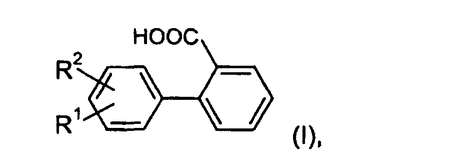 worin R¹ und R² die in der Beschreibung und den Ansprüchen genannte Bedeutung haben können.

### Hintergrund der Erfindung

Biphenyl-2-carbonsäuren der Formel (I) haben als Zwischenprodukte eine hohe Bedeutung bei der Herstellung von pharmazeutisch interessanten Wirkstoffen, insbesondere bei der Herstellung von Pharmawirkstoffen, die als Angiotensin-II-Antagonisten Verwendung finden können.

Verfahren zur Herstellung von Biphenyl-2-carbonsäure und deren Derivaten (I) sind aus dem Stand der Technik bekannt. Ein für den Hintergrund der Erfindung wesentlicher Zugang erfolgt gemäß der von Meyers et al. (z.B. *Tetrahedron* (1985) Vol. 41, 837-860) beschriebenen Kupplung von aromatischen Grignard-Verbindungen (II) mit gegebenenfalls substituierten (2-Methoxyphenyl)-2-oxazolinen (III) gemäß Schema 1, wobei zunächst die entsprechenden (2-Oxazolinyl)-2-biphenyl-Derivate (IV) erhalten werden.

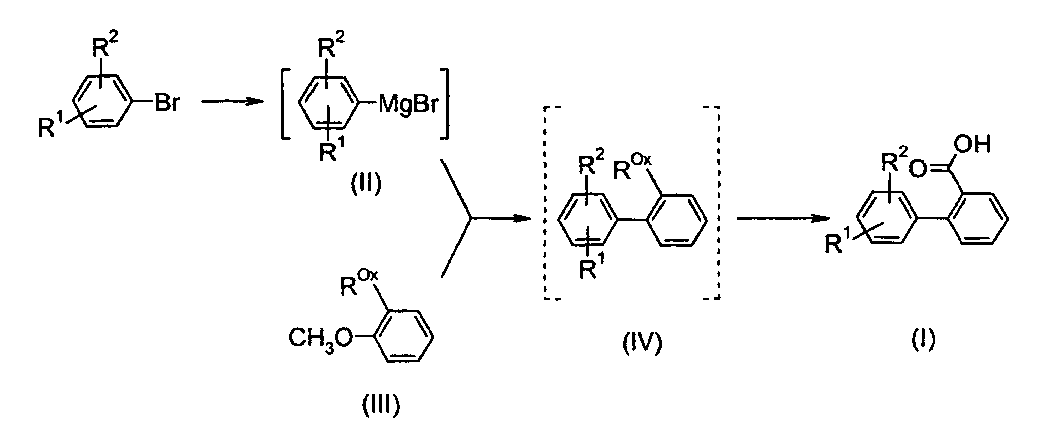

### Schema 1:

Dabei steht der Rest R^{Ox} für einen gegebenenfalls substituierten Oxazolin-2-yl-Rest. Die Definition der Reste R¹ und R² ist dem späteren Teil der Beschreibung sowie den Ansprüchen zu entnehmen. Durch Verseifung der Oxazoline (IV) gelingt die Umwandlung in die entsprechenden Carbonsäuren der Formel (I). Diese Verseifung von (IV) kann bei formaler Betrachtung über zwei verschiedene Reaktionswege verlaufen. In Schema 2 sind diese Reaktionswege exemplarisch für die Herstellung von Biphenyl-2-carbonsäure, ausgehend von am Oxazolin-Rest unsubstituierten Biphenyl-oxazolin dargestellt (d.h. R¹ und R² = Wasserstoff; R^{Ox} = Oxazolin-2-yl).

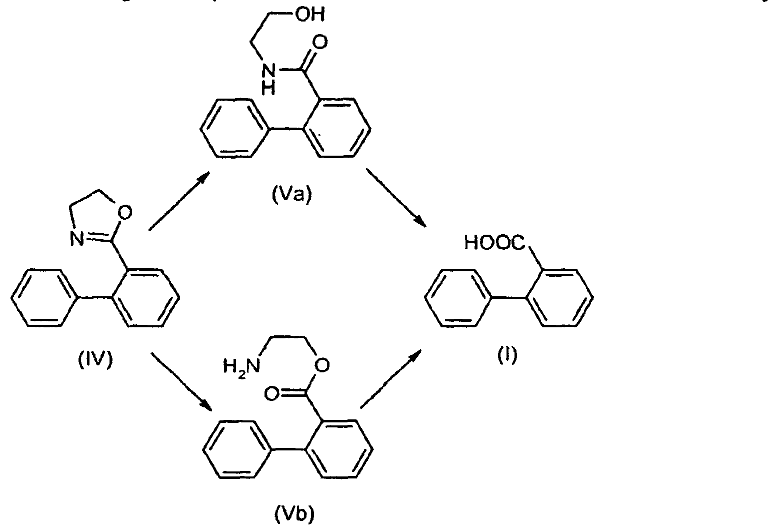

### Schema 2:

Die Verseifung des Oxazolins gemäß im Stand der Technik bekannten Reaktionsbedingungen führt im ersten Schritt zur Bildung des Aminoesters (Vb) (Meyers et al. *J. Org.* Chem. (1974) Vol. 39, 2787-2793). Der Aminoester (Vb) kann dann in einer zweiten Reaktionsstufe, z. B. durch mehrstündiges Kochen in 10 - 25 %iger Natronlauge zur Carbonsäure (I) verseift werden.

Für ein großtechnisches Herstellverfahren ist es allerdings wünschenswert, den Verseifungsprozess in Form eines Eintopfverfahrens durchzuführen.
Die in Anlehnung an die aus dem Stand der Technik bekannten Verfahren durchgeführte saure Verseifung im Eintopf-Verfahren (z.B. gemäß EP59983) führte jedoch bei großtechnischer Durchführung nur zu unbefriedigenden Ergebnissen.

Es wurde beobachtet, daß aufgrund der geringen Löslichkeit in den gemäß dem Stand der Technik einzusetzenden Lösemitteln (z.B. wässrige Salzsäure gemäß EP 59983) Aminoester (Vb) nach seiner Bildung teilweise ausfällt. Es kommt zum Niederschlag von (Vb) am Rührwerk sowie an den Wänden des Reaktionsgefäßes. Dies führt dazu, daß Aminoesters (Vb) sukzessive der Reaktionslösung entzogen wird und aufgrund der geringen Löslichkeit dann praktisch nicht mehr zur Weiterreaktion zur gewünschten Zielverbindung (I) zur Verfügung steht. Eine weitergehende Ausbeutenreduzierung erfolgt aufgrund des Einschlusses von Produkt (I) im auskristallisierten und regelrecht verklumpenden Aminoester (Vb).

Die vorstehend genannten Nachteile führen zu einem erhöhten Mehraufwand bei de großtechnischen Herstellung von (I), da im Rahmen der Aufarbeitung und Reinigung des Endprodukts zum einen eine Abtrennung des Aminoesters (Vb) erfolgen muß, zum anderen für die Umsetzung von ausgefallenem Aminoester (Vb) zum Endprodukt ein seperater Syntheseschritt zu erfolgen hat.

Es ist daher Ziel und Aufgabe der vorliegenden Erfindung, ein großtechnisch anwendbares Verfahren zur Herstellung von Derivaten/Homologen der Biphenyl-2-carbonsäure bereitzustellen, welches die bei den aus dem Stand der Technik bekannten Verfahren auftretenden Nachteile überwindet.

### Detaillierte Beschreibung der Erfindung

Überraschenderweise wurde gefunden, daß die bei aus dem Stand der Technik bekannten Herstellungsverfahren für Biphenyl-2-carbonsäure-Derivate auftretenden Nachteile vermieden werden können, wenn die Verseifung des Oxazolins (IV) mit Salzsäure bei erhöhter Temperatur unter Druck, in Gegenwart eines mit Wasser nicht mischbaren, inerten, organischen Lösemittels erfolgt.

Die vorliegende Erfindung zielt folglich auf ein großtechnisch anwendbares Herstellverfahren für Biphenyl-2-carbonsäure-Derivate der allgemeinen Formel (I) 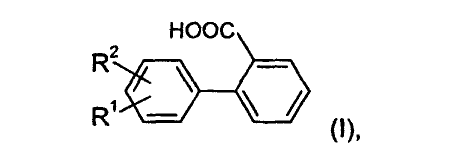 worin
- R¹ und R²: gleich oder verschieden, Wasserstoff, C₁-C₆-Alkyl, welches gegebenenfalls durch Halogen substituiert sein kann, C₁-C₆-Alkoxy, C₁- C₆-Acyl, C₁-C₆-Alkoxycarbonyl, COOH, Phenyl, Benzyl, Halogen,
Hydroxy, Nitro oder Amino bedeuten, oder worin R¹ und R² gemeinsam mit benachbarten Kohlenstoffatomen des Phenylrings einen gesättigten oder ungesättigten 5- oder 6-gliedrigen Carbocyclus bilden, der gegebenenfalls durch C₁-C₄-Alkyl, Halogen, COOH, Phenyl oder Hydroxy substituiert sein kann;
dadurch gekennzeichnet, daß ein (2-Oxazolinyl)-2-biphenyl-Derivat der allgemeinen Formel (IV) 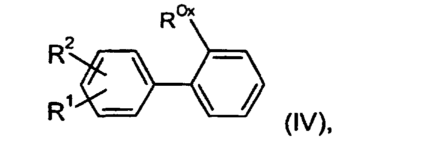 worin
- R¹ und R²: die vorstehend genannte Bedeutung tragen und
- R^{Ox}: für einen Oxazolin-2-yl-Rest steht, der gegebenenfalls ein-, zwei-, dre oder vierfach substituiert sein kann durch einen oder mehrere der Reste C₁-C₆-Alkyl, welches gegebenfalls durch Halogen, Hydroxy oder C₁-C₄-Alkoxy substituiert sein kann, C₁-C₆-Alkoxy, Phenyl, welches gegebenenfalls durch C₁-C₄-Alkyl, C₁-C₄-Alkoxy, Hydroxy, Nitro oder Amino substituiert sein kann, Benzyl, Pyridyl oder C₁-C₆- Alkoxycarbonyl,
mit Salzsäure bei erhöhter Temperatur unter Druck, in Gegenwart eines mit Wasser nicht mischbaren, inerten, organischen Lösemittels verseift wird.

Erfindungsgemäß bevorzugt ist ein Verfahren zur Herstellung von Biphenyl-2-carbonsäure-Derivaten der allgemeinen Formel (I), worin
- R¹ und R²: gleich oder verschieden, Wasserstoff, C₁-C₄-Alkyl, welches gegebenenfalls durch Fluor, Chlor oder Brom substituiert sein kann, C₁- C₄-Alkoxy, C₁-C₄-Acyl, C₁-C₄-Alkoxycarbonyl, COOH, Phenyl, Benzyl, Fluor, Chlor, Brom, Hydroxy, Nitro oder Amino bedeuten, oder worin R¹ und R² gemeinsam mit benachbarten Kohlenstoffatomen des Phenylrings einen ungesättigten 6-gliedrigen Carbocyclus bilden, der gegebenenfalls durch C₁-C₄-Alkyl, Fluor, Chlor, Brom, COOH, Phenyl oder Hydroxy substituiert sein kann;
dadurch gekennzeichnet, daß ein (2-Oxazolinyl)-2-biphenyl-Derivat der allgemeinen Formel (IV), worin
- R¹ und R²: die vorstehend genannte Bedeutung tragen und
- R^{Ox}: für einen Oxazolin-2-yl-Rest steht, der gegebenenfalls ein- oder zweifach substituiert sein kann durch einen oder mehrere der Reste C₁- C₄-Alkyl, welches gegebenfalls durch Fluor, Chlor, Brom, Hydroxy oder C₁-C₄-Alkoxy substituiert sein kann, C₁-C₄-Alkoxy, Phenyl, welches gegebenenfalls durch C₁-C₄-Alkyl, C₁-C₄-Alkoxy, Hydroxy, Nitro oder Amino substituiert sein kann, Benzyl oder C₁-C₄-Alkoxycarbonyl,
mit Salzsäure bei erhöhter Temperatur unter Druck, in Gegenwart eines mit Wasser nicht mischbaren, inerten, organischen Lösemittels verseift wird.

Besonders bevorzugt ist ein Verfahren zur Herstellung von Biphenyl-2-carbonsäure-Derivaten der allgemeinen Formel (I), worin
- R¹ und R²: gleich oder verschieden, Wasserstoff, Methyl, Ethyl, n-Propyl, iso- Propyl, n-Butyl, tert-Butyl, CF₃, Methoxy, Ethoxy, COOH, Phenyl, Benzyl, Fluor, Chlor, Brom, Hydroxy, Nitro oder Amino bedeuten, oder worin
R¹ und R² gemeinsam mit benachbarten Kohlenstoffatomen des Phenylrings einen anellierten Phenylring bilden, der gegebenenfalls durch Methyl, Ethyl, n-Propyl, iso-Propyl, tert.-Butyl, Fluor, Chlor, Brom, COOH, Phenyl oder Hydroxy substituiert sein kann,
dadurch gekennzeichnet, daß ein (2-Oxazolinyl)-2-biphenyl-Derivat der allgemeinen Formel (IV), worin
- R¹ und R²: die vorstehend genannte Bedeutung tragen und
- R^{Ox}: für einen Oxazolin-2-yl-Rest steht, der gegebenenfalls ein- oder zweifach substituiert sein kann durch einen oder mehrere der Reste Methyl, Ethyl, n-Propyl, iso-Propyl, n-Butyl, tert.-Butyl, Methoxymethyl, Hydroxymethyl, Methoxy, Ethoxy, Phenyl, welches gegebenenfalls durch Methyl, Ethyl, n-Propyl, iso-Propyl, n-Butyl, tert.-Butyl, Methoxy, Ethoxy oder Hydroxy substituiert sein kann, Benzyl, Methoxycarbonyl oder Ethoxycarbonyl,
mit Salzsäure bei erhöhter Temperatur unter Druck, in Gegenwart eines mit Wasser nicht mischbaren, inerten, organischen Lösemittels verseift wird.

Ferner ist erfindungsgemäß von Bedeutung ein Verfahren zur Herstellung von Biphenyl-2-carbonsäure-Derivaten der allgemeinen Formel (I), worin
- R¹ und R²: gleich oder verschieden, Wasserstoff, Methyl, CF₃, COOH, Phenyl, Fluor oder Hydroxy bedeuten, oder worin
R¹ und R² gemeinsam mit benachbarten Kohlenstoffatomen des Phenylrings einen anellierten Phenylring bilden,
dadurch gekennzeichnet, daß ein (2-Oxazolinyl)-2-biphenyl-Derivat der allgemeinen Formel (IV), worin
- R¹ und R²: die vorstehend genannte Bedeutung tragen und
- R^{Ox}: für einen Oxazolin-2-yl-Rest steht, der gegebenenfalls ein- oder zweifach substituiert sein kann durch einen oder mehrere der Reste Methyl, Ethyl, Methoxy, Ethoxy, Phenyl oder Benzyl,
mit Salzsäure bei erhöhter Temperatur unter Druck, in Gegenwart eines mit Wasser nicht mischbaren, inerten, organischen Lösemittels verseift wird.

Von besonderer Bedeutung ist ein Verfahren zur Herstellung von Biphenyl-2-carbonsäure-Derivaten der allgemeinen Formel (I), worin
R¹ und R² gleich oder verschieden, Wasserstoff, Methyl oder CF₃ bedeuten, dadurch gekennzeichnet, daß ein (2-Oxazolinyl)-2-biphenyl-Derivat der allgemeinen Formel (IV), worin
- R¹ und R²: die vorstehend genannte Bedeutung tragen und
- R^{Ox}: für einen Oxazolin-2-yl-Rest steht, der gegebenenfalls ein- oder zweifach durch Methyl substituiert sein kann,
mit Salzsäure bei erhöhter Temperatur unter Druck, in Gegenwart eines mit Wasser nicht mischbaren, inerten, organischen Lösemittels verseift wird.

In erfindungsgemäß besonders bevorzugter Art und Weise wird folgt vorgegangen. In einem geeignet dimensionierten Reaktionsgefäß werden pro Mol Oxazolin-2-ylbiphenyl (IV) 0.08-0.8, bevorzugt 0.15-0.5, besonders bevorzugt ca. 0.2 I Wasser und 3.0-6.0 Mol, bevorzugt 3.5 - 5.0 Mol, besonders bevorzugt ca. 4.0 Mol Salzsäure eingebracht. Bevorzugt erfolgt die Zugabe der vorstehend genannten Salzsäure in Form wässriger Lösungen, besonders bevorzugt in Form 36.5%-iger wässriger Lösung, so daß eine Salzsäurekonzentration 20 - 30 %, besonders bevorzugt von ca 24 % resultiert.

Nach Inertisieren mit Schutzgas, bevorzugt mit Stickstoff, wird das Reaktionsgefäß evakuiert (auf ca. 50 mbar) und pro Mol eingesetzte Ausgangsverbindung (IV) mit 0.05-0.2, bevorzugt 0.08-0.15, besonders bevorzugt mit ca 0.1 I eines inerten organischen Lösemittels versetzt. Als inerte organische Lösemittel kommen erfindungsgemäß aliphatische oder aromatische Kohlenwasserstoffe und aromatische Chlorkohlenwasserstoffe mit 6-10 C-Atomen in Betracht. Bevorzugt sind aliphatische oder aromatische Kohlenwasserstoffe mit 7-8 C-Atomen. Als erfindungsgemäß verwendbare Lösemittel seien als bevorzugt genannt Toluol, Xylol,Chlorbenzol und Methylcyclohexan. Besonders bevorzugt ist Methylcyclohexan.

Nach Zugabe des inerten organischen Lösemittels wird die Reaktionslösung auf eine Temperatur im Bereich von 120-160°C, bevorzugt 130-150°C besonders bevorzugt 140-145°C, erhitzt. Bei konstanter Temperatur wird weitere 3-10 h, bevorzugt 4-8 Stunden gerührt. Die Apparatur wird dabei verschlossen (im Betrieb beispielsweise durch Schließen der Brüdenabsperrklappe), sodaß sich durch das vorstehend genannte Erhitzen der Reaktionslösung innerhalb der Apparatur ein Innendruck von 3-6 barÜ (= bar Überdruck), bevorzugt von 4-5 barÜ einstellt. Je nach Siedepunkt des eingesetzten Lösemittels kann die Temperatur variiert werden, damit sich vorstehend genannter Innendruck einstellt. Hierdurch ergibt sich der erfindungsgemäß weitere Vorteil, daß konventionelle Apparaturen wie beispielsweise DIN-Emaille-Apparturen (Druckstufe 6 barÜ) Verwendung finden können.

Anschließend erfolgt die Abkühlung des Reaktionsgefäßes auf eine Temperatur, bei der die Apparatur maximal Atmosphärendruck aufweist (20 - 50°C). Ein Unterdruck wird gegebenenfalls mit Inertgas ausgeglichen. Das Reaktionsgemisch wird zur Aufarbeitung mit einem geeigneten Lösemittel oder Lösemittelgemisch versetzt, das die Abtrennung der salzsauren Wasserphase ohne Produktverluste ermöglicht. Bevorzugt ist die Verwendung von Toluol, Xylol oder Methylcyclohexan in Mischungen mit Tetrahydrofuran. Besonders bevorzugt ist eine Mischung von Toluol und Tetrahydrofuran im Verhältnis von etwa 1:1. Pro Mol eingesetzte Ausgangsverbindung (IV) werden zwischen 0.1-1 I des vorstehend genannten organischen Lösemittels oder Lösemittelgemischs eingebracht. Bevorzugt werden pro mol eingesetztes Oxazolin (IV) 0.2-0.5 I des vorstehend genannten organischen Lösemittels oder Lösemittelgemischs verwendet. Besonders bevorzugt werden pro Mol eingesetztes Oxazolin (IV) ca. 0.3 - 0.35 I des organischen Lösemittels oder Lösemittelgemischs eingebracht.

Die wässrige Unterphase wird anschließend abgetrennt und die verbleibende Oberphase nochmals mehrfach, bevorzugt 2-3-fach, besonders bevorzugt 2-fach mit Wasser extrahiert. Die Menge eingesetzten Waschwassers liegt erfindungsgemäß pro Extraktionsvorgang in einem Bereich von 0.05-0.5 I Wasser pro Mol eingesetzten Oxazolins (IV). Bevorzugt finden je Extraktionsschritt 0.1-0.2 I Wasser pro eingesetztes Mol Ausgangsverbindung (IV) Verwendung.

Die so gewaschene organische Oberphase wird anschließend alkalisch gestellt. Hierzu können erfindungsgemäß wässrige Lösungen der Alkali- oder Erdalkalihydroxide eingesetzt werden. Bevorzugt kommen wässrige Lösungen des Lithium-, Natrium- oder Kaliumhydroxids zum Einsatz. Als Base erfindungsgemäß besonders bevorzugt ist wässrige Natriumhydroxidlösung. Pro Mol Ausgangsverbindung (IV) werden 0.7-1 Mol Base, bevorzugt 0.8-0.9 Mol Base eingesetzt.
Die Unterphase wird nach erfolgter Phasentrennung in ein weiteres Reaktionsgefäß abgelassen. Die verbleibende Oberphase wird daraufhin erneut der vorstehend genannten Alkalisierung unterworfen. Dabei werden erfindungsgemäß allerdings nur noch ca. 10% w/w der im ersten Alkalisierungschritt eingesetzten Basenmenge zugesetzt. Nach erneuter Abtrennung der Unterphase werden die vereinigten wässrigen Extrakte destillativ von mitgerissenem Lösemittel befreit. Pro Mol eingesetzte Ausgangsverbindung (IV) werden etwa 0.05-0.5l Wasser, bevorzugt zwischen 0.07 und 0.2 I, besonders bevorzugt ca. 0.1 I Wasser abdestilliert. Nach Abkühlen auf eine Temperatur unterhalb von 40°C, bevorzugt auf eine Temperatur im Bereich von 20-30°C, besonders bevorzugt auf 25°C, werden pro Mol eingesetzte Ausgangsverbindung 0.1-0.5 I, bevorzugt ca. 0.2 I Wasser gegeben und anschließend mit 1-5 Mol, bevorzugt, 2-4 Mol, besonders bevorzugt ca. 3.5 Mol Salzsäure sauer gestellt.
Das ausgefallene Produkt wird abgeschleudert, mit Wasser gewaschen und getrocknet.

Die nachfolgenden Beispiele dienen der Illustration exemplarisch durchgeführter, erfindungsgemäßer Syntheseverfahren zur Herstellung von Derivaten der Biphenyl-2-carbonsäure der allgemeinen Formel (I). Sie sind lediglich als mögliche, beispielhaft dargestellte Vorgehensweisen zu verstehen, ohne die Erfindung auf deren Inhalt zu beschränken.

### Beispiel 1

In einem 1200 I-Email-Rührwerksapparat werden 265 kg 4'-Methyl-2-(4,4-dimethyloxazolin-2-yl)biphenyl, 205 I Wasser und 400 kg 36,5 %ige Salzsäure eingebracht. Nach Inertisieren mit Stickstoff wird auf ca. 50 mbar evakuiert und dann 102,5 I Methylcyclohexan zugegeben. Nach Schließen der Brüdenabsperrklappe wird innerhalb etwa 1 h auf ca. 140°C erhitzt und noch weitere 4 bis 8 h bei 140 - 145 °C gerührt. Dabei stellt sich ein Innendruck von 4 - 5 barÜ ein. Danach wird auf 20 - 30°C abgekühlt, mit Stickstoff auf Atmosphärendruck eingestellt und 175 I Toluol und 150 I THF zugegeben. Die wäßrige Unterphase wird abgetrennt und die verbleibende organische Oberphase nochmals mit 205 I und dann mit 103 I Wasser extrahiert. Zur Oberphase werden weitere 512 I Wasser und 80 kg Natronlauge 45 % zugegeben und die Unterphase nach Absitzen in einen weiteren 1200 I-Email-Rührwerksapparat abgelassen. Diese Operation wird mit 103 I Wasser und 8,9 kg Natronlauge 45 % wiederholt.
Aus den vereinigten wäßrigen Extrakten werden zunächst ca. 103 I abdestilliert und nach Abkühlen auf 25°C 205 I Wasser und dann 97 kg 36,5 %ige Salzsäure zugegeben. Das Produkt wird abgeschleudert, mit Wasser gewaschen und getrocknet. Ausbeute : 190 kg 4'-Methylbiphenyl-2-carbonsäure (90%)

### Beispiel 2

In einem 1200 I-Email-Rührwerksapparat werden 251 kg 2-(4,4-Dimethyloxazolin-2-yl)biphenyl, 205 I Wasser und 400 kg 36,5 %ige Salzsäure eingebracht. Nach Inertisieren mit Stickstoff wird auf ca. 50 mbar evakuiert und dann 102,5 I Methylcyclohexan zugegeben. Nach Schließen der Brüdenabsperrklappe wird innerhalb etwa 1 h auf ca. 140°C erhitzt und noch weitere 4 bis 8 h bei 140 - 145 °C gerührt. Dabei stellt sich ein Innendruck von 4 - 5 barÜ ein. Danach wird auf 20 - 30°C abgekühlt, mit Stickstoff auf Atmosphärendruck eingestellt und 175 I Toluol und 150 I THF zugegeben. Die wäßrige Unterphase wird abgetrennt und die verbleibende organischen Oberphase nochmals mit 205 I und dann mit 103 I Wasser extrahiert. Zur Oberphase werden weitere 512 I Wasser und 80 kg Natronlauge 45 % zugegeben und die Unterphase nach Absitzen in einen weiteren 1200 I-Email-Rührwerksapparat abgelassen. Diese Operation wird mit 103 I Wasser und 8,9 kg Natronlauge 45 % wiederholt. Aus den vereinigten wäßrigen Extrakten werden zunächst ca. 103 I abdestilliert und nach Abkühlen auf 25°C 205 I Wasser und dann 97 kg 36,5 %ige Salzsäure zugegeben. Das Produkt wird abgeschleudert, mit Wasser gewaschen und getrocknet.
Ausbeute : 180 kg Biphenyl-2-carbonsäure (91%)

### Vergleichsbeispiel :

In einem 1200 I-Email-Rührwerksapparat werden 265 kg 4'-Methyl-2-(4,4-dimethyloxazolin-2-yl)biphenyl, 2051 Wasser und 400 kg 36,5 %ige Salzsäure eingebracht. Nach Inertisieren mit Stickstoff, Evakuieren auf ca. 50 mbar und Schließen der Brüdenabsperrklappe wird der Apparateinhalt innerhalb etwa 1 h auf ca. 140°C erhitzt und noch weitere 4 bis 8 h bei 140 - 145 °C gerührt, wobei sich ein Innendruck von 4 - 5 barÜ einstellt. Danach wird auf 20 - 30°C abgekühlt, mit Stickstoff auf Atmosphärendruck eingestellt und 1751 Toluol und 150 I THF zugegeben. Die wäßrige Unterphase wird zum Abwasser gegeben und die verbleibende organischen Oberphase nochmals mit 205 1 und dann mit 103 I Wasser extrahiert. Zur Oberhase werden weitere 512 I Wasser und 80 kg Natronlauge 45 % zugegeben und die Unterphase nach Absitzen in einen weiteren 1200 I-Email-Rührwerksapparat abgelassen. Diese Operation wird mit 103 I Wasser und 8,9 kg Natronlauge 45 % wiederholt. Aus den vereinigten wäßrigen Extrakten werden zunächst ca. 103 I abdestilliert und nach Abkühlen auf 25°C 205 I Wasser und dann 97 kg 36,5 %ige Salzsäure zugegeben. Das Produkt wird abgeschleudert, mit Wasser gewaschen und getrocknet.
Ausbeute : 100 kg 4'-Methylbiphenyl-2-carbonsäure (47%)

## Patentansprüche

1. Herstellverfahren für Biphenyl-2-carbonsäure-Derivate der allgemeinen Formel (I) 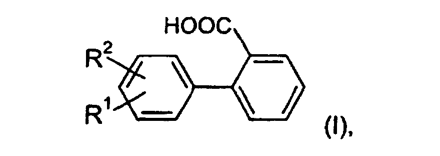 worin
R¹ und R² gleich oder verschieden, Wasserstoff, C₁-C₆-Alkyl, welches gegebenenfalls durch Halogen substituiert sein kann, C₁-C₆-Alkoxy, C₁- C₆-Acyl, C₁-C₆-Alkoxycarbonyl, COOH, Phenyl, Benzyl, Halogen, Hydroxy, Nitro oder Amino bedeuten, oder worin
R¹ und R² gemeinsam mit benachbarten Kohlenstoffatomen des Phenylrings einen gesättigten oder ungesättigten 5- oder 6-gliedrigen Carbocyclus bilden, der gegebenenfalls durch C₁-C₄-Alkyl, Halogen, COOH, Phenyl oder Hydroxy substituiert sein kann;
**dadurch gekennzeichnet, daß** ein (2-Oxazolinyl)-2-biphenyl-Derivat der allgemeinen Formel (IV) 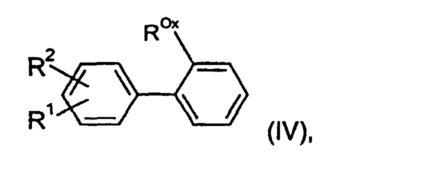 worin
R¹ und R² die vorstehend genannte Bedeutung tragen und
R^{Ox} für einen Oxazolin-2-yl-Rest steht, der gegebenenfalls ein-, zwei-, drei- oder vierfach substituiert sein kann durch einen oder mehrere der Reste C₁-C₆-Alkyl, welches gegebenfalls durch Halogen, Hydroxy oder C₁-C₄-Alkoxy substituiert sein kann, C₁-C₆-Alkoxy, Phenyl, welches gegebenenfalls durch C₁-C₄-Alkyl, C₁-C₄-Alkoxy, Hydroxy, Nitro oder Amino substituiert sein kann, Benzyl, Pyridyl oder C₁-C₆- Alkoxycarbonyl,
mit Salzsäure bei einer Temperatur von 120-160°C und einem Druck von 3-6 barÜ in Gegenwart eines mit Wasser nicht mischbaren, inerten, organischen Lösemittels verseift wird.

2. Verfahren gemäß Anspruch 1, worin
R¹ und R² gleich oder verschieden, Wasserstoff, C₁-C₄-Alkyl, welches gegebenenfalls durch Fluor, Chlor oder Brom substituiert sein kann, C₁- C₄-Alkoxy, C₁-C₄-Acyl, C₁-C₄-Alkoxycarbonyl, COOH, Phenyl, Benzyl, Fluor, Chlor, Brom, Hydroxy, Nitro oder Amino bedeuten, oder worin
R¹ und R² gemeinsam mit benachbarten Kohlenstoffatomen des Phenylrings einen ungesättigten 6-gliedrigen Carbocyclus bilden, der gegebenenfalls durch C₁-C₄-Alkyl, Fluor, Chlor, Brom, COOH, Phenyl oder Hydroxy substituiert sein kann, und
R^{Ox} für einen Oxazolin-2-yl-Rest steht, der gegebenenfalls ein- oder zweifach substituiert sein kann durch einen oder mehrere der Reste C₁- C₄-Alkyl, welches gegebenfalls durch Fluor, Chlor, Brom, Hydroxy oder C₁-C₄-Alkoxy substituiert sein kann, C₁-C₄-Alkoxy, Phenyl, welches gegebenenfalls durch C₁-C₄-Alkyl, C₁-C₄-Alkoxy, Hydroxy, Nitro oder Amino substituiert sein kann, Benzyl oder C₁-C₄-Alkoxycarbonyl.

3. Verfahren gemäß Anspruch 1 oder 2, worin
R¹ und R² gleich oder verschieden, Wasserstoff, Methyl, Ethyl, n-Propyl, iso- Propyl, n-Butyl, tert-Butyl, CF₃, Methoxy, Ethoxy, COOH, Phenyl, Benzyl, Fluor, Chlor, Brom, Hydroxy, Nitro oder Amino bedeuten, oder worin
R¹ und R² gemeinsam mit benachbarten Kohlenstoffatomen des Phenylrings einen anellierten Phenylring bilden, der gegebenenfalls durch Methyl, Ethyl, n-Propyl, iso-Propyl, tert.-Butyl, Fluor, Chlor, Brom, COOH, Phenyl oder Hydroxy substituiert sein kann, und
R^{Ox} für einen Oxazolin-2-yl-Rest steht, der gegebenenfalls ein- oder zweifach substituiert sein kann durch einen oder mehrere der Reste Methyl, Ethyl, n-Propyl, iso-Propyl, n-Butyl, tert.-Butyl, Methoxymethyl, Hydroxymethyl, Methoxy, Ethoxy, Phenyl, welches gegebenenfalls durch Methyl, Ethyl, n-Propyl, iso-Propyl, n-Butyl, tert.-Butyl, Methoxy, Ethoxy oder Hydroxy substituiert sein kann, Benzyl, Methoxycarbonyl oder Ethoxycarbonyl.

4. Verfahren gemäß einem der Ansprüche 1 bis 3, worin
R¹ und R² gleich oder verschieden, Wasserstoff, Methyl, CF₃, COOH, Phenyl, Fluor oder Hydroxy bedeuten, oder worin
R¹ und R² gemeinsam mit benachbarten Kohlenstoffatomen des Phenylrings einen anellierten Phenylring bilden, und
R^{Ox} für einen Oxazolin-2-yl-Rest steht, der gegebenenfalls ein- oder zweifach substituiert sein kann durch einen oder mehrere der Reste Methyl, Ethyl, Methoxy, Ethoxy, Phenyl oder Benzyl.

5. Verfahren gemäß einem der Ansprüche 1 bis 4, worin
R¹ und R² gleich oder verschieden, Wasserstoff, Methyl oder CF₃ bedeuten,
und
R^{Ox} für einen Oxazolin-2-yl-Rest steht, der gegebenenfalls ein- oder zweifach durch Methyl substituiert sein kann.

6. Verfahren gemäß einem der Ansprüche 1 bis 5, **dadurch gekennzeichnet, daß** bei einem Druck von 4-5 barÜ verseift wird.

7. Verfahren gemäß einem der Ansprüche 1 bis 6, **dadurch gekennzeichnet, daß** pro Mol eingesetzter Ausgangsverbindung der allgemeinen Formel (IV) 3.0 - 6.0 Mol Salzsäure zur Verseifung eingesetzt werden.

8. Verfahren gemäß einem der Ansprüche 1 bis 7, **dadurch gekennzeichnet, daß** pro Mol eingesetzter Ausgangsverbindung der allgemeinen Formel (IV) 3.5 - 5.0 Mol Salzsäure zur Verseifung eingesetzt werden.

9. Verfahren gemäß einem der Ansprüche 1 bis 8, **dadurch gekennzeichnet, daß** als organisches Lösemittel aliphatische oder aromatische Kohlenwasserstoffe oder aromatische Chlorkohlenwasserstoffe mit 6-10 C-Atomen verwendet werden.

10. Verfahren gemäß einem der Ansprüche 1 bis 9, **dadurch gekennzeichnet, daß** als organisches Lösemittel aliphatische oder aromatische Kohlenwasserstoffe mit 7-8 C-Atomen oder Chlorbenzol verwendet werden.

11. Verfahren gemäß einem der Ansprüche 1 bis 10, **dadurch gekennzeichnet, daß** als organisches Lösemittel Toluol, Xylol, Chlorbenzol und Methylcyclohexan verwendet wird.

12. Verfahren gemäß einem der Ansprüche 1 bis 11, **dadurch gekennzeichnet, daß** als organisches Lösemittel Methylcyclohexan verwendet wird.

13. Verfahren zur Herstellung von 4'-Methylbiphenyl-2-carbonsäure gemäß einem der Ansprüche 1 bis 12.

## Claims

1. Process for preparing biphenyl-2-carboxylic acid derivatives of general formula (I) 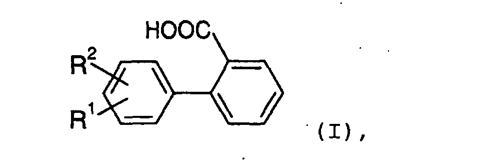 wherein
R¹ and R² which may be identical or different denote hydrogen, C₁-C₆-alkyl, which may optionally be substituted by halogen, C₁-C₆-alkoxy, C₁-C₆-acyl, C₁-C₆-alkoxycarbonyl, COOH, phenyl, benzyl, halogen, hydroxy, nitro or amino, or wherein
R¹ and R² together with adjacent carbon atoms of the phenyl ring form a saturated or unsaturated 5- or 6-membered carbocyclic group which may optionally be substituted by C₁-C₄-alkyl, halogen, COOH, phenyl or hydroxy;
**characterised in that** a (2-oxazolinyl)-2-biphenyl derivative of general formula (IV) 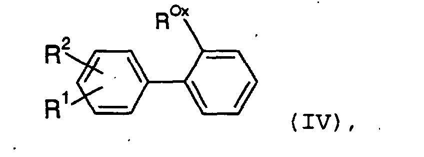 wherein
R¹ and R² are as hereinbefore defined and
R^{Ox} denotes an oxazolin-2-yl group, which may optionally be mono-, di-, tri- or tetra-substituted by one or more of the groups C₁-C₆-alkyl, which may optionally be substituted by halogen, hydroxy or C₁-C₄-alkoxy, C₁-C₆-alkoxy, phenyl, which may optionally be substituted by C₁-C₄-alkyl, C₁-C₄-alkoxy, hydroxy, nitro or amino, or benzyl, pyridyl or C₁-C₆-alkoxycarbonyl,
is saponified with hydrochloric acid at a temperature of 120-160°C under a pressure of 3-6 baro, in the presence of an inert organic solvent which is immiscible with water.

2. Process according to claim 1, wherein
R¹ and R², which may be identical or different, denote hydrogen, C₁-C₄-alkyl, which may optionally be substituted by fluorine, chlorine or bromine, C₁-C₄-alkoxy, C₁-C₄-acyl, C₁-C₄-alkoxycarbonyl, COOH, phenyl, benzyl, fluorine, chlorine, bromine, hydroxy, nitro or amino, or wherein
R¹ and R² together with adjacent carbon atoms of the phenyl ring form an unsaturated 6-membered carbocyclic group which may optionally be substituted by C₁-C₄-alkyl, fluorine, chlorine, bromine, COOH, phenyl or hydroxy, and
R^{Ox} denotes an oxazolin-2-yl group, which may optionally be mono- or disubstituted by one or more of the groups C₁-C₄-alkyl, which may optionally be substituted by fluorine, chlorine, bromine, hydroxy or C₁-C₄-alkoxy, C₁-C₄-alkoxy, phenyl, which may optionally be substituted by C₁-C₄-alkyl, C₁-C₄-alkoxy, hydroxy, nitro or amino, benzyl or C₁-C₄-alkoxycarbonyl.

3. Process according to claim 1 or 2, wherein
R¹ and R², which may be identical or different, denote hydrogen, methyl, ethyl, n-propyl, isopropyl, n-butyl, tert-butyl, CF₃, methoxy, ethoxy, COOH, phenyl, benzyl, fluorine, chlorine, bromine; hydroxy, nitro or amino, or wherein
R¹ and R² together with adjacent carbon atoms of the phenyl ring form an anellated phenyl ring which may optionally be substituted by methyl, ethyl, n-propyl, isopropyl, tert.-butyl, fluorine, chlorine, bromine, COOH, phenyl or hydroxy,
and
R^{Ox} denotes an oxazolin-2-yl group, which may optionally be mono- or disubstituted by one or more of the groups methyl, ethyl, n-propyl, isopropyl, n-butyl, tert.butyl, methoxymethyl, hydroxymethyl, methoxy or ethoxy, or phenyl, which may optionally be substituted by methyl, ethyl, n-propyl, isopropyl, n-butyl, tert.-butyl, methoxy, ethoxy or hydroxy, or benzyl, methoxycarbonyl or ethoxycarbonyl.

4. Process according to one of claims 1 to 3, wherein R¹ and R², which may be identical or different, denote hydrogen, methyl, CF₃, COOH, phenyl, fluorine or hydroxy, or wherein
R¹ and R² together with adjacent carbon atoms of the phenyl ring form an anellated phenyl ring, and
R^{Ox} denotes an oxazolin-2-yl group, which may optionally be mono- or disubstituted by one or more of the groups methyl, ethyl, methoxy, ethoxy, phenyl or benzyl.

5. Process according to one of claims 1 to 4, wherein R¹ and R², which may be identical or different, denote hydrogen, methyl or CF₃, and
R^{Ox} denotes an oxazolin-2-yl group which may optionally be mono- or disubstituted by methyl.

6. Process according to one of claims 1 to 5, **characterised in that** saponification is carried out at a pressure of 4-5 baro.

7. Process according to one of claims 1 to 6, **characterised in that** 3.0 - 6.0 mol of hydrochloric acid are used for the saponification per mol of starting compound of general formula (IV) used.

8. Process according to one of claims 1 to 7, **characterised in that** 3.5 - 5.0 mol of hydrochloric acid are used for the saponification per mol of starting compound of general formula (IV) used.

9. Process according to one of claims 1 to 8, **characterised in that** aliphatic or aromatic hydrocarbons or aromatic chlorohydrocarbons with 6-10 carbon atoms are used as the organic solvent.

10. Process according to one of claims 1 to 9, **characterised in that** aliphatic or aromatic hydrocarbons with 7-8 carbon atoms or chlorobenzene are used as the organic solvent.

11. Process according to one of claims 1 to 10, **characterised in that** toluene, xylene, chlorobenzene and methylcyclohexane are used as the organic solvent.

12. Process according to one of claims 1 to 11, **characterised in that** methylcyclohexane is used as the organic solvent.

13. Process for preparing 4'-methylbiphenyl-2-carboxylic acid according to one of claims 1 to 12.

## Revendications

1. Procédé de préparation de dérivés d'acide biphényl-2-carboxylique de formule générale (I) 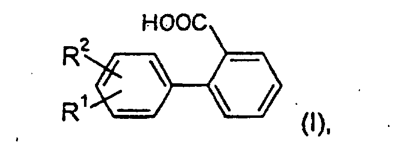 où
R¹ et R², identiques ou différents, représentent l'hydrogène, alkyle en C₁-C₆, qui peut éventuellement être substitué par halogène, alcoxy en C₁-C₆, acyle en
C₁-C₆, alcoxy en C₁-C₆-carbonyle, COOH, phényle, benzyle, halogène, hydroxyle, nitro ou amino, ou bien où
R¹ et R² forment avec des atomes de carbone voisins du cycle phényle un carbocycle à 5 ou 6 chaînons saturé ou insaturé, qui peut éventuellement être substitué par alkyle en C₁-C₄, halogène, COOH, phényle ou hydroxyle ;
**caractérisé en ce qu'**un dérivé de (2-oxazolinyl)-2-biphényle de formule générale (IV) 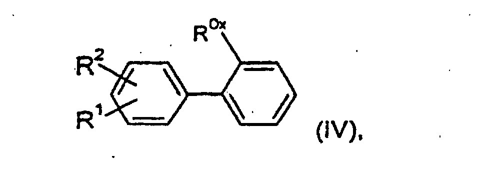 où
R¹ et R² ont la signification citée précédemment et
R^{Ox} représente un reste oxazolin-2-yle qui peut éventuellement être substitué une, deux, trois ou quatre fois par un ou plusieurs des restes alkyle en C₁-C₆, qui peut éventuellement être substitué par halogène, hydroxyle ou alcoxy en C₁-C₄, alcoxy en C₁-C₆, phényle, qui peut éventuellement être substitué par alkyle en C₁-C₄, alcoxy en C₁-C₄, hydroxyle, nitro ou amino, benzyle, pyridyle ou alcoxy en C₁-C₆-carbonyle, est saponifié avec l'acide chlorhydrique à une température de 120-160°C et une pression de 3-6 bar man. en présence d'un solvant organique inerte non miscible à l'eau.

2. Procédé selon la revendication 1 où R¹ et R², identiques ou différents, représentent l'hydrogène, alkyle en C₁-C₄, qui peut éventuellement être substitué par le fluor, le chlore ou le brome, alcoxy en C₁-C₄, acyle en C₁-C₄, alcoxy en C₁-C₄-carbonyle, COOH, phényle, benzyle, fluor, chlore, brome, hydroxyle, nitro ou amino, ou bien où
R¹ et R² forment avec des atomes de carbone voisins du cycle phényle un carbocycle à 6 chaînons insaturé, qui peut éventuellement être substitué par alkyle en C₁-C₄, fluor, chlore, brome, COOH, phényle ou hydroxyle ; et R^{Ox} représente un reste oxazolin-2-yle qui peut éventuellement être substitué une ou deux fois par un ou plusieurs des restes alkyle en C₁-C₄, qui peut éventuellement être substitué par fluor, chlore, brome, hydroxyle ou alcoxy en C₁-C₄, alcoxy en C₁-C₄, phényle, qui peut éventuellement être substitué par alkyle en C₁-C₄, alcoxy en C₁-C₄, hydroxyle, nitro ou amino, benzyle ou alcoxy en C₁-C₄-carbonyle.

3. Procédé selon la revendication 1 ou 2 où R¹ et R², identiques ou différents, représentent l'hydrogène, méthyle, éthyle, n-propyle, isopropyle, n-butyle, tert-butyle, CF₃, méthoxy, éthoxy, COOH, phényle, benzyle, fluor, chlore, brome, hydroxyle, nitro ou amino, ou bien où
R¹ et R² forment avec des atomes de carbone voisins du cycle phényle un cycle phényle condensé, qui peut éventuellement être substitué par méthyle, éthyle, n-propyle, isopropyle, tert-butyle, fluor, chlore, brome, COOH, phényle ou hydroxyle ; et
R^{Ox} représente un reste oxazolin-2-yle qui peut éventuellement être substitué une ou deux fois par un ou plusieurs des restes méthyle, éthyle, n-propyle, isopropyle, n-butyle, tert-butyle, méthoxyméthyle, hydroxyméthyle, méthoxy, éthoxy, phényle, qui peut éventuellement être substitué par méthyle, éthyle, n-propyle, isopropyle, n-butyle, tert-butyle, méthoxy, éthoxy ou hydroxyle, benzyle, méthoxycarbonyle ou éthoxycarbonyle.

4. Procédé selon l'une des revendications 1 à 3 où
R¹ et R², identiques ou différents, représentent l'hydrogène, méthyle, CF₃, COOH, phényle, fluor ou hydroxyle, ou bien où
R¹ et R² forment avec des atomes de carbones voisins du cycle phényle un cycle phényle condensé, et
R^{Ox} représente un reste oxazolin-2-yle qui peut éventuellement être substitué une ou deux fois par un ou plusieurs des restes méthyle, éthyle, méthoxy, éthoxy, phényle ou benzyle.

5. Procédé selon l'une des revendications 1 à 4 où
R¹ et R², identiques ou différents, représentent l'hydrogène, méthyle ou CF₃, et
R^{Ox} représente un reste oxazolin-2-yle qui peut éventuellement être substitué une ou deux fois par méthyle.

6. Procédé selon l'une des revendications 1 à 5 **caractérisé en ce que** l'on saponifie à une pression de 4-5 bar man.

7. Procédé selon l'une des revendications 1 à 6 **caractérisé en ce que** l'on utilise par mole de composé de départ de formule générale (IV) utilisé 3,0 - 6,0 moles d'acide chlorhydrique pour la saponification.

8. Procédé selon l'une des revendications 1 à 7 **caractérisé en ce que** l'on utilise par mole de composé de départ de formule générale (IV) utilisé 3,5 - 5,0 moles d'acide chlorhydrique pour la saponification.

9. Procédé selon l'une des revendications 1 à 8 **caractérisé en ce que** l'on utilise comme solvant organique des hydrocarbures aliphatiques ou aromatiques ou des hydrocarbures chlorés aromatiques ayant 6-10 atomes de carbone.

10. Procédé selon l'une des revendications 1 à 9 **caractérisé en ce que** l'on utilise comme solvant organique des hydrocarbures aliphatiques ou aromatiques ayant 7-8 atomes de carbone ou le chlorobenzène.

11. Procédé selon l'une des revendications 1 à 10 **caractérisé en ce que** l'on utilise comme solvant organique le toluène, le xylène, le chlorobenzène et le méthylcyclohexane.

12. Procédé selon l'une des revendications 1 à 11 **caractérisé en ce que** l'on utilise le méthylcyclohexane comme solvant organique.

13. Procédé de préparation de l'acide 4'-méthylbiphényl-2-carboxylique selon l'une des revendications 1 à 12.
